Europäisches Patentamt

European Patent Office

Office européen des brevets

(19)

(11) EP 0 749 754 A2

(12) DEMANDE DE BREVET EUROPEEN

(43) Date de publication:
27.12.1996 Bulletin 1996/52

(51) Int Cl.6: **A61K 49/00**
// A61K31:07, A61K31:19,
A61K31:38

(21) Numéro de dépôt: 96401169.6

(22) Date de dépôt: 31.05.1996

(84) Etats contractants désignés:
CH DE FR GB LI

(30) Priorité: 19.06.1995 FR 9507301

(71) Demandeur: CENTRE INTERNATIONAL DE
RECHERCHES DERMATOLOGIQUES
GALDERMA - CIRD GALDERMA
F-06565 Valbonne (FR)

(72) Inventeurs:
• Demarchez, Michel
06620 Le Bar sur Loup (FR)
• Jomard, André
06460 Saint Vallier de Thiey (FR)

(74) Mandataire: Andral, Christophe André Louis
L'OREAL
Centre de Recherche Charles Zviak
Département Propriété Industrielle
90, rue du Général Roguet
92583 Clichy Cedex (FR)

(54) **Procédé pour identifier des composés agonistes des récepteurs RXRs**

(57) La présente invention concerne un procédé pour identifier des molécules agonistes des RXRs, caractérisé en ce qu'il comprend les étapes suivantes : (i) on applique topiquement sur une partie de la peau d'un mammifère une quantité suffisante d'un composé qui est un ligand d'au moins un récepteur de la superfamille des récepteurs stéroïdiens/thyroïdiens, autre qu'un ligand spécifique des récepteurs RXRs, et pouvant s'hétérodimériser avec les RXRs, (ii) on administre par voie systémique ou topique sur ce même mammifère ou sur cette même partie de la peau du mammifère, avant, pendant ou après l'étape (i), une molécule susceptible de présenter une activité agoniste des RXRs et (iii) on évalue la réponse sur la partie de la peau ainsi traitée du mammifère.

## Description

La présente invention a pour objet un procédé pour identifier des composés agonistes des récepteurs RXRs en utilisant un mammifère, tel que les rongeurs (rat, cobaye, hamster, lapin, souris...).

On sait que l'acide rétinoïque *tout-trans* est un puissant modulateur (i.e. un inhibiteur ou, au contraire, un stimulateur, selon la nature des cellules traitées) de la différenciation et de la prolifération de nombreux types cellulaires normaux ou transformés. Par exemple, il inhibe la différenciation des cellules épithéliales, tels que les kératinocytes de l'épiderme. Il inhibe aussi la prolifération de nombreuses cellules transformées telles que les cellules de mélanomes.

On sait, d'une manière générale, que l'acide rétinoïque *tout-trans (all-trans* retinoic acid) agit sur la différenciation et la prolifération des cellules en interagissant avec des récepteurs nucléaires appelés RARs (Retinoic Acid Receptors) contenus dans le noyau cellulaire. Ces récepteurs, après fixation du ligand (i.e. de l'acide rétinoïque *tout-trans),* interagissent avec la région promotrice de gènes régulés par l'acide rétinoïque au niveau d'éléments de réponses spécifiques. Pour se fixer sur les éléments de réponse, les RARs s'hétérodimèrisent avec un autre type de récepteurs connus sous le nom de RXRs, le ligand naturel des RXRs étant l'acide 9-*cis*-rétinoïque. Les RXRs sont considérés comme des "master regulatory proteins" car ils interagissent pour former des hétéromères, comme avec les RARs, avec d'autres membres de la superfamille des récepteurs stéroïdiens/thyroïdiens, tels que le récepteur de la vitamine D3 (VDR), le récepteur de la triiodothyroxine (TR) et les PPARs (Peroxisome Proliferator Activated Receptors).

Compte tenu des activités biologiques mentionnés ci-dessus, on comprend l'intérêt de trouver de nouveaux composés agonistes des récepteurs RXRs. Ainsi, il a déjà été décrit dans de nombreux brevets et publications l'intérêt d'utiliser ces composés dans le domaine pharmaceutique, et plus particulièrement dermatologique.

De nombreux analogues structuraux synthétiques de l'acide rétinoïque tout-*trans* ou de l'acide 9-*cis*-rétinoïque, couramment dénommés "rétinoïdes", ont été décrits à ce jour dans la littérature. Certaines de ces molécules sont capables de se fixer et d'activer (agonistes) ou à l'inverse de désactiver (antagoniste) spécifiquement les RARs ou, au contraire, les RXRs. D'autres analogues, enfin, ne présentent aucune sélectivité particulière vis-à-vis de ces différents récepteurs. A cet égard, et par exemple, l'acide 9-*cis* rétinoïque active à la fois les RARs et les RXRs, sans sélectivité notable pour l'un ou l'autre de ces récepteurs (ligand non spécifique), alors que l'acide rétinoïque *tout-trans* active, quant à lui, sélectivement les RARs (agoniste spécifique des RARs).

Chez la souris, il a été montré que l'acide rétinoïque *tout-trans* en administration unique par voie topique induit une réponse, dépendante de la dose, de prolifération épidermique, avec un maximum de réponse quatre jours après l'application ("Retinoïc acid provokes a regeneration-like proliferative response in murine epidermis" Arch. Dermatol. Res. 1992, 284:418-423). En accord avec ce résultat, la réponse à une application topique d'acide rétinoïque *tout-trans* sur l'oreille de souris se traduit notamment par une augmentation de l'épaisseur de l'oreille de souris. Cette augmentation de l'épaisseur de l'oreille de souris semble être due à une augmentation de l'épaisseur de l'épiderme et à une apparition d'un oedème dermique. Cette réponse peut donc être facilement mesurée à l'aide d'un appareil, tel que l'oditest, celle-ci étant maximale aux cinquième et sixième jours après l'application.

A l'inverse, dans les mêmes conditions opératoires, les ligands spécifiques des RXRs appliqués seuls de manière topique ou orale sur l'oreille de souris n'induisent aucun effet clinique.

La Demanderesse vient de découvrir que la réponse à une application topique sur la peau d'un mammifère d'un composé qui est un ligand actif d'au moins un récepteur de la superfamille des récepteurs stéroïdiens/thyroïdiens, autre qu'un ligand spécifique des récepteurs RXRs, et pouvant s'hétérodimériser avec les RXRs peut être synergisée par l'administration par voie systémique ou topique d'un agoniste des RXRs.

Ainsi, la présente invention a pour but de proposer un procédé simple pour identifier des molécules agonistes des RXRs.

Ce but et d'autres sont atteints par la présente invention qui concerne un procédé pour identifier des molécules agonistes des RXRs, caractérisé en ce qu'il comprend les étapes suivantes : (i) on applique topiquement sur une partie de la peau d'un mammifère une quantité suffisante d'au moins un composé qui est un ligand actif d'au moins un récepteur de la superfamille des récepteurs stéroïdiens/thyroïdiens, autre qu'un ligand spécifique des récepteurs RXRs, et pouvant s'hétérodimériser avec les RXRs, (ii) on administre par voie systémique ou topique sur ce même mammifère ou sur cette même partie de la peau du mammifère, avant, pendant ou après l'étape (i), une molécule susceptible de présenter une activité agoniste des RXRs et (iii) on évalue la réponse sur la partie de la peau ainsi traitée du mammifère et on la compare avec la réponse obtenue sur cette même partie de peau traitée par la seule étape (i).

Ainsi lorsque la molécule administrée est un agoniste des RXRs, l'épaisseur de la partie de la peau traitée du mammifère par une molécule qui est un ligand actif d'au moins un récepteur de la superfamille des récepteurs stéroïdiens/thyroïdiens, autre qu'un ligand spécifique des récepteurs RXRs, et pouvant s'hétérodimériser avec les RXRs augmente. On peut alors parler de synergie de la réponse, puisque la molécule susceptible de présenter une activité agoniste des RXRs à tester n'induit aucun effet clinique lorsqu'elle est seule appliquée par voie topique.

De manière pratique, le mammifère est un rongeur tel qu'une souris, un rat, un cobaye, un hamster ou un lapin.

La partie de la peau de mammifère utilisée peut être n'importe quelle partie du corps du mammifère.

La réponse sur la partie de la peau ainsi traitée du mammifère à évaluer correspond à une modification clinique de celle-ci. De manière générale, cette réponse à évaluer correspond à une modification de l'épaisseur de la partie de la peau ainsi traitée.

Ainsi, la mesure de l'épaisseur de la partie de la peau ainsi traitée peut être réalisée par toute méthode connue en soi.

Lorsque la partie de la peau utilisée est lisse, on peut mesurer son épaisseur en la pliant.

De manière plus pratique, on utilise la peau de l'oreille. La mesure de l'épaisseur de l'oreille peut alors être réalisée par un oditest.

Bien entendu, l'évaluation de l'étape (iii) correspond à une mesure de la réponse de la partie de la peau ainsi traitée et à une comparaison de cette mesure avec celle de la réponse de cette même partie de la peau traitée, dans les mêmes conditions, avec le même ligand actif d'au moins un récepteur de la superfamille des récepteurs stéroïdiens/ thyroïdiens, autre qu'un ligand spécifique des récepteurs RXRs, pouvant s'hétérodimériser avec les RXRs seul.

Parmi les ligands actifs d'au moins un récepteur de la superfamille des récepteurs stéroïdiens/thyroïdiens, autre qu'un ligand spécifique des récepteurs RXRs, pouvant s'hétérodimériser avec les RXRs, on peut notamment citer les ligands des RARs (Retinoic Acid Receptors), du VDR (Vitamine D3 Receptor), des PPARs (Peroxisome Proliferator Activated Receptor) et du TR (récepteur de la triiodothyroxine).

On préfère utiliser comme ligands actifs d'au moins un récepteur de la superfamille des récepteurs stéroïdiens/ thyroïdiens, autre qu'un ligand spécifique des récepteurs RXRs, pouvant s'hétérodimériser avec les RXRs les agonistes des RARs.

Parmi les agonistes des RARs, on peut plus particulièrement citer :

- l'acide rétinoïque *tout-trans,*
- l'acide 2-(5,6,7,8-tetrahydro-5,5,8,8-tetraméthyl-2-naphtyl)-6-benzo[b]thiophen carboxylique,
- l'acide 4-[(5,6,7,8-tétrahydro-5,5,8,8-tétraméthyl-2-naphtyl) carboxamido] benzoïque,
- l'acide 4-[(5,6,7,8-tétrahydro-5,5,8,8-tétraméthyl-2-naphtyl) carbamoyl] benzoïque.

Comme exemples de ligands du récepteur VDR, on peut citer les dérivés de la vitamine D suivants :

- $1\alpha,25$-dihydroxyvitamine D3,
- $1\alpha$-hydroxyvitamine D3,
- 25-hydroxyvitamine D3,
- $1\alpha,25$-dihydroxyvitamine D2,
- $1\alpha,24$-dihydroxyvitamine D2.

Parmi les ligands spécifiques des récepteurs PPARs, on peut notamment citer l'acide bromopalmitique et ses analogues.

Dans ce qui suit ou ce qui précède, on entend par voie topique, toute technique d'administration d'un produit par application directe de ce dernier sur une partie superficielle (ou externe) du corps, et par voie systémique, toute technique d'administration d'un produit par une voie autre que topique, par exemple entérale et/ou parentérale. Dans le cas de la voie systémique, on préfère utiliser la voie orale.

La quantité suffisante d'au moins un ligand actif d'au moins un récepteur de la superfamille des récepteurs stéroïdiens/thyroïdiens, autre qu'un ligand spécifique des récepteurs RXRs, pouvant s'hétérodimériser avec les RXRs à appliquer correspond à celle à laquelle on observe une réponse oedémateuse de la partie traitée de la peau du mammifère après les étapes (i) et (ii) (c'est à dire après application des deux composés). De manière pratique, cette quantité suffisante correspond à la limite maximale à laquelle on n'observe pas encore de réponse de la partie traitée de la peau du mamifère après l'étape (i) (c'est à dire après la seule application du ligand actif d'au moins un récepteur de la superfamille des récepteurs stéroïdiens/thyroïdiens, autre qu'un ligand spécifique des récepteurs RXRs, pouvant s'hétérodimériser avec les RXRs). Ainsi, de préférence et selon la nature du composé utilisé, cette quantité varie entre 0,0001% et 5% en poids par volume de solution appliquée.

On va maintenant donner, à titre nullement limitatif, plusieurs exemples destinés d'une part à démontrer les effets attachés à la présente invention, et, d'autre part, à illustrer diverses formulations concrètes conformes à l'invention.

## EXEMPLE 1

Le test utilisé est donc celui de l'oedème de l'oreille de souris induit par application topique de l'acide 2-(5,6,7,8-tetrahydro-5,5,8,8-tetraméthyl-2-naphtyl)-6-benzo[b]thiophen carboxylique (analogue de l'acide rétinoïque *tout-trans)* à 0,003% en poids par volume. Selon ce modèle, une application topique de l'acide 2-(5,6,7,8-tetrahydro-5,5,8,8-tetraméthyl-2naphtyl)-6-benzo[b]thiophen carboxylique sur l'oreille provoque une inflammation qui se caractérise par l'augmentation de l'épaisseur de cette oreille, cette augmentation devenant maximale au bout de 5 jours après l'application. La réponse peut donc être quantifiée par une mesure de l'épaisseur de l'oreille par un oditest.

Selon ce modèle, dans une étude préliminaire, la dose maximale à appliquer d'acide 2-(5,6,7,8-tetrahydro-5,5,8,8-tetraméthyl-2-naphtyl)-6-benzo[b]thiophen carboxylique n'induisant pas d'augmentation de l'épaisseur de l'oreille de souris a été déterminée. C'est cette dose qui est utilisée lors du protocole opératoire suivant.

Le protocole opératoire exact est le suivant : 10 souris sont tout d'abord traitées avec l'acide 2-(5,6,7,8-tetrahydro-5,5,8,8-tetraméthyl-2-naphtyl)-6-benzo[b]thiophen carboxylique (composé A), en procédant sur l'une de leur oreille à une application topique à un temps t=0 avec 20 µl d'une solution d'acétone comprenant 0,003 % en poids par volume du composé A. On fait absorber par voie orale à 5 (= groupe 2) sur 10 des souris ainsi traitées de l'acide 4-[(3,5,5,8,8-pentaméthyl-5,6,7,8-tétrahydro-2-naphtyl)carbonyl] benzoïque (composé B) dans de l'huile de type cremophor (EL 25%) à partir de t=0 et ceci une fois par jour pendant 11 jours. Les 5 souris n'ayant pas absorbé le composé B constituent le groupe 1. La réponse est quantifiée par une mesure de l'épaisseur de l'oreille à t = 5 ou 6 jours. Les résultats sont ensuite exprimés en % de l'effet de synergie calculé de la façon suivante :

$$\frac{\text{épaisseur oreille souris (Groupe 2) - épaisseur oreille souris (Groupe 1)}}{\text{épaisseur oreille souris (Groupe 1)}} \times 100$$

Par ailleurs, le composé B est décrit comme étant un agoniste spécifique des RXRs (voir : Marcus F. Boehm et al., J. Med. Chem. 1994, 37, 2930-2941).

Les résultats obtenus sont rassemblés dans le tableau 1 suivant.

tableau 1

| voie topique | dose (%P/V) | voie orale | dose (mg/kg) | effet de synergie (%) |
|---|---|---|---|---|
| composé A | 0,003 | composé B | 0 | 0 |
| composé A | 0,003 | composé B | 10 | 73 |
| composé A | 0,003 | composé B | 30 | 118 |
| PN signifie poids par volume. | | | | |

De plus, dans les mêmes conditions opératoires, l'application par voie topique de ligand spécifique des RXRs seul, tel que le composé B, n'induit aucune réponse.

Ainsi, on démontre clairement grâce à ce test que l'association d'un ligand de récepteurs RARs avec un composé connu comme étant une molécule agoniste RXR augmente de manière importante la réponse comparativement à la réponse induite par une application topique unique d'un ligand de récepteurs RARs.

EXEMPLE 2

On réalise exactement le même test qu'à l'exemple 1, sauf que le composé B, au lieu d'être administré oralement, est apppliqué topiquement dans une solution d'acétone à une dose de 0,1 % en poids par volume sur une oreille déjà traitée par le composé A à une concentration de 0,01% ou de 0,003 % P/V.

Les résultats obtenus sont rassemblés dans le tableau 2 suivant.

tableau 2

| voie topique | dose (%P/V) | voie topique | dose (% P/V) | effet de synergie (%) |
|---|---|---|---|---|
| composé A | 0,01 | composé B | 0,1 | 159 |
| composé A | 0,003 | composé B | 0,1 | 541 |
| PV signifie poids par volume. | | | | |

EXEMPLE 3

On réalise exactement le même test qu'à l'exemple 1, sauf que le composé B est remplacé par un composé C : l'acide (E)-2-[2-(3,5,5,8,8-pentaméthyl-5,6,7,8-tétrahydro-2-naphtyl)-1-propényl]4-thiophènecarboxylique et est appliqué de manière topique comme dans l'exemple 2.

Le composé C est décrit comme étant un agoniste spécifique des RXRs dans la demande de brevet WO 94/17796 déposée par la société Allergan.

Les résultats obtenus sont rassemblés dans le tableau 3 suivant.

tableau 3

| voie topique | dose (%P/V) | voie topique | dose (% P/V) | effet de synergie (%) |
|---|---|---|---|---|
| composé A | 0,01 | composé C | 0,1 | 169 |
| composé A | 0,003 | composé C | 0,1 | 617 |
| PV signifie poids par volume. | | | | |

**Revendications**

1. Procédé pour identifier des molécules agonistes des RXRs, caractérisé en ce qu'il comprend les étapes suivantes : (i) on applique topiquement sur une partie de la peau d'un mammifère une quantité suffisante d'au moins un composé qui est un ligand actif d'au moins un récepteur de la superfamille des récepteurs stéroïdiens/thyroïdiens, autre qu'un ligand spécifique des récepteurs RXRs, et pouvant s'hétérodimériser avec les RXRs, (ii) on administre par voie systémique ou topique sur ce même mammifère ou sur cette même partie de la peau du mammifère, avant, pendant ou après l'étape (i), une molécule susceptible de présenter une activité agoniste des RXRs et (iii) on évalue la réponse sur la partie de la peau ainsi traitée du mammifère et on la compare avec la réponse obtenue sur cette même partie de peau traitée par la seule étape (i).

2. Procédé selon la revendication précédente, caractérisé en ce que le mammifère est un rongeur tel qu'une souris, un rat, un cobaye, un hamster ou un lapin.

3. Procédé selon l'une des revendications précédentes, caractérisé en ce que la partie de la peau de mammifère utilisée est la peau de l'oreille.

4. Procédé selon l'une des revendications précédentes, caractérisé en ce que la réponse à évaluer à l'étape (iii) correspond à une modification de l'épaisseur de la partie de la peau ainsi traitée du mammifère.

5. Procédé selon l'une quelconque des revendications précédentes, caractérisé en ce que le ligand actif d'au moins un récepteur de la superfamille des récepteurs stéroïdiens/thyroïdiens, autre qu'un ligand spécifique des récepteurs RXRs, et pouvant s'hétérodimériser avec les RXRs est choisi parmi les ligands des RARs (Retinoic Acid Receptors), du VDR (Vitamine D3 Receptor) et des PPARs (Peroxisome Proliferator Activated Receptors) et du TR (récepteur de la triiodothyroxine).

6. Procédé selon la revendication précédente, caractérisé en ce que le ligand actif d'au moins un récepteur de la superfamille des récepteurs stéroïdiens/thyroïdiens, autre qu'un ligand spécifique des récepteurs RXRs, et pouvant s'hétérodimériser avec les RXRs, est choisi parmi les agonistes des RARs.

7. Procédé selon la revendication précédente, caractérisé en ce que l'agoniste des RARs est choisi parmi :

   - l'acide rétinoïque *tout-trans,*
   - l'acide 2-(5,6,7,8-tetrahydro-5,5,8,8-tetraméthyl-2-naphtyl)-6-benzo[b]thiophen carboxylique,
   - l'acide 4-[(5,6,7,8-tétrahydro-5,5,8,8-tétraméthyl-2-naphtyl) carboxamido] benzoïque,
   - l'acide 4-[(5,6,7, 8-tétrahydro-5,5,8,8-tétraméthyl-2-naphtyl) carbamoyl] benzoïque.

8. Procédé selon la revendication 5, caractérisé en ce que le ligand du récepteur VDR est choisi parmi :

   - $1\alpha$,25-dihydroxyvitamine D3,
   - $1\alpha$-hydroxyvitamine D3,
   - 25-hydroxyvitamine D3,
   - $1\alpha$,25-dihydroxyvitamine D2,
   - $1\alpha$,24-dihydroxyvitamine D2.

9. Procédé selon la revendication 5, caractérisé en ce que le ligand des récepteurs PPARs est l'acide bromopalmitique.

**10.** Procédé selon l'une quelconque des revendications précédentes, caractérisé en ce que cas la voie systémique est la voie orale.